Europäisches Patentamt

European Patent Office

Office européen des brevets

(11) Veröffentlichungsnummer: **0 125 541**
B1

# EUROPÄISCHE PATENTSCHRIFT

(45) Veröffentlichungstag der Patentschrift:
18.01.89

(51) Int. Cl.⁴: **G 01 N 9/26**, G 01 N 33/44

(21) Anmeldenummer: 84104687.3

(22) Anmeldetag: 26.04.84

(54) Verfahren und Vorrichtung zur Messung der Gasbeladung einer flüssigen Kunststoffkomponente.

(30) Priorität: 13.05.83 DE 3317486
04.10.83 DE 3336037

(43) Veröffentlichungstag der Anmeldung:
21.11.84 Patentblatt 84/47

(45) Bekanntmachung des Hinweises auf die Patenterteilung:
18.01.89 Patentblatt 89/3

(84) Benannte Vertragsstaaten:
DE FR GB IT SE

(56) Entgegenhaltungen:
EP-A- 0 022 946
DE-A- 2 142 865
GB-A- 1 032 490
GB-A- 1 071 798
US-A- 4 089 206
US-A- 4 329 869

(73) Patentinhaber: Krauss-Maffei Aktiengesellschaft,
Krauss-Maffei-Strasse 2, D-8000 München 50 (DE)

(72) Erfinder: Hölzl, Emil, Dipl.-Ing.,
Nikolaus-Rüdiger-Strasse 25, D-8000 München 50 (DE)
Erfinder: Grglc, Ivica, Dipl.-Ing., Forstenrieder Allee 24,
D-8000 München 71 (DE)
Erfinder: Söchtig, Wolfgang, Dipl.-Ing., Finkenstrasse 1,
D-8034 Germering (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf eine Vorrichtung und ein Verfahren zur Messung der Gasbeladung einer unter dem Systemdruck einer Anlage zur Herstellung von Schaumkunststoff, insbesondere einer Polyurethan-Schäumanlage stehenden, flüssigen Kunststoffkomponente, bei dem aus einem Tank in periodischen Zeitabständen eine Probemenge in ein Messgefäss abzweigbar ist, in dem mittels eines Überlaufs eine bestimmte Höhe $h$ einer aus flüssiger und gasbeladener Kunststoffkomponente bestehenden Flüssigkeitssäule einstellbar ist, und in dem eine den hydrostatischen Druck $p$ der Flüssigkeitssäule als Mass für die Dichte $\rho$ der gasbeladenen Kunststoffkomponente wertende Dichtemesseinrichtung angeordnet ist.

Aus der US-PS 4 089 206 sind ein Verfahren und eine Vorrichtung dieser Art bekannt, bei dem das Messgefäss als Dekompressionskammer dient und der Messwert über den Anstieg der Flüssigkeitssäule aufgrund einer Dekompression erhalten wird. Dieses Messverfahren arbeitet verhältnismässig träge und ermöglicht insbesondere bei mit Feststoffen, wie z.B. Glasfasern, beladenen flüssigen Kunststoffen nur geringe Messgenauigkeit, da die sich verändernde Füllstandshöhe weder mit dem Auge noch mit Füllstandsmessgeräten mit vertretbarem Aufwand in der erforderlichen Genauigkeit und Schnelligkeit erfassbar ist. Auch wird dabei lediglich ein relativer, kein absoluter Dichtewert gemessen.

Aus der DE-AS 2 142 865, der GB-PS 1 071 798 sowie der GB-PS 1 032 490 sind ferner Vorrichtungen bekannt, mit denen mit Hilfe der Messung des hydrostatischen Drucks einer Flüssigkeitssäule mit vorbestimmter Höhe die Dichte und somit der Grad der Beladung der Flüssigkeit mit anderen Komponenten, beispielsweise mit gasförmigem Kohlendioxid gemessen werden kann.

Der Erfindung liegt die Aufgabe zugrunde eine Vorrichtung und ein Verfahren zu schaffen, womit das Messgefäss in einfacher Weise und in schneller Folge mit frischer flüssiger Kunststoffkomponenten gespült werden kann und die Messung in jedem beliebigen Druckzustand vom Niveau des Systemdrucks bis zum Atmosphärendruck vorgenommen werden kann.

Diese Aufgabe wird sowohl durch die Merkmale des Kennzeichens des Anspruchs 1 als auch durch die Merkmale des Kennzeichens des Anspruchs 6 gelöst.

Die Lösung nach Anspruch 1 hat darüberhinaus den Vorteil, dass durch die Anordnung des steuerbaren Kolbens auch der Gasbeladungsgrad einer flüssigen Kunststoffkomponente bei Unterdruck gemessen werden kann.

Besonders wertvoll ist jedoch die Möglichkeit im Messgefäss ständig und schnell erneuerbare Mengen von flüssiger Kunststoffkomponente bei Atmosphärendruck messen zu können, da sich die Herstellerangaben über die spezifischen Eigenschaften, insbesondere das Gasbeladungsvermögen der Kunststoffkomponenten, in der Regel auf den Zustand bei Atmopshärendruck beziehen.

Dieser Zustand entspricht auch den Verarbeitungsbedingungen in den Formwerkzeugen, beispielsweise einer Polyurethan-Schäumanlage.

Zweckmässigerweise besteht die Dichtemesseinrichtung aus einem am Boden oder der Seitenwand des Messgefässes angeordneten Druckmessfühler, beispielsweise einer Bourdon Messdose, mit der die Messwerte exakt und verzögerungsfrei erhalten werden können.

Vorzugsweise ist die Dichtemesseinrichtung mit Einrichtungen zur Kompensation des über der Flüssigkeitssäule herrschenden, vom atmosphärischen Druck abweichenden Druckniveaus ausgerüstet, wodurch sich die Dichtewerte der gasbeladenen Flüssigkeit bei jedem beliebigen Druckzustand ermitteln lassen.

In einer bevorzugten Ausführungsform ist der Dichtemesseinrichtung ein Rechner nachgeschaltet, in dem mit Hilfe einiger Anfangsmesswerte und im Rechner eingespeicherter Bezugswerte, beispielsweise empirisch ermittelter Eichwerte und/oder einer physikalischen Gesetzmässigkeiten entsprechenden mathematischen Funktion, der Dichtewert der gasbeladenen Kunststoffkomponente vor dem Abschluss der Entspannungsphase vorbestimmbar ist. Diese Massnahme trägt besonders zur Verkürzung des Messvorgangs bei.

Eine weitere Massnahme zur Beschleunigung des Messvorgangs kann darin bestehen, die Steuereinheit mit einem, den Öffnungsvorgang des Entlüftungsventils verzögernden Zeitschaltglied zu versehen, wodurch bei abgesperrten Ein- und Auslassventilen und abgesenkten Kolben die Flüssigkeitssäule einem Unterdruck aussetzbar ist. Zur Verminderung des gerätetechnischen Aufwand kann die Messvorrichtung gemäss den Merkmalen aus Anspruch 8 ausgestaltet sein.

Die Erfindung wird nachfolgend an einem Ausführungsbeispiel anhand der Zeichnung erläutert, die in schematischer Darstellung in

Fig. 1 eine Polyurethan-Schäumanlage mit einer Vorrichtung zur Messung der Gasbeladung einer flüssigen Kunststoffkomponente und in

Fig. 2 eine weitere Ausführungsform der Messvorrichtung zeigt.

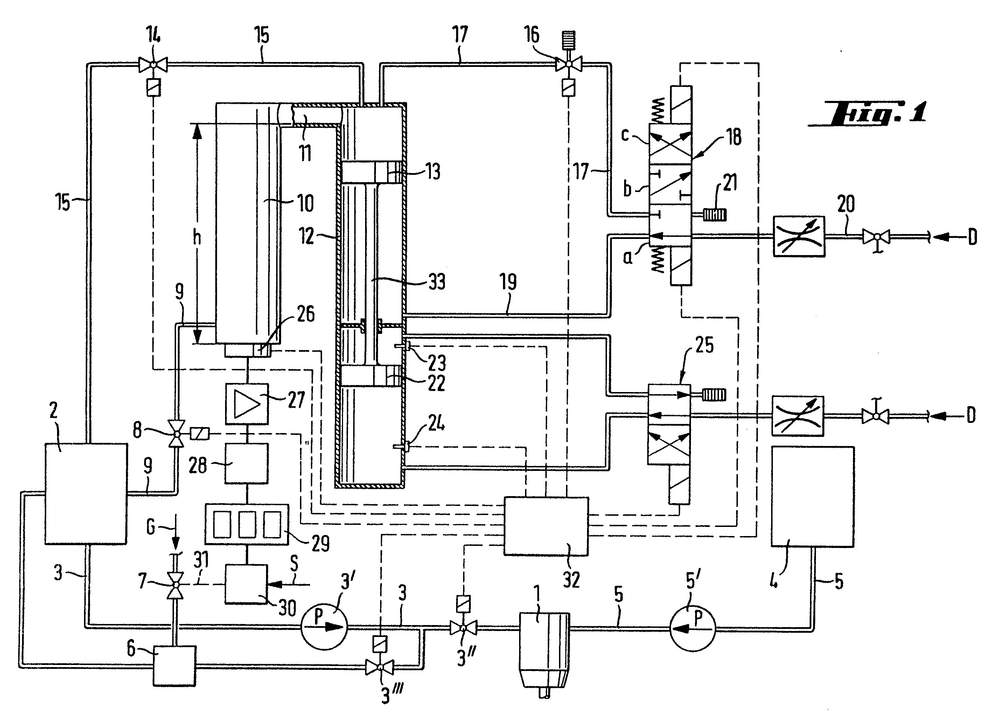

Die Polyurethan-Schäumanlage nach Fig. 1 weist einen Mischkopf 1 auf, dem aus einem Polyoltank 2 über eine Polyolleitung 3 mittels einer Polyolpumpe 3' die Kunststoffkomponenten Polyol und dem aus einem Isocyanattank 4 über eine Isocyanat-Leitung 5 mittels einer Isocyanatpumpe 5' die Kunststoffkomponente Isocyanat zuführbar ist. Das in der Polyol-Leitung 3 geförderte Polyol ist gesteuert von Ventilen 3'' und 3''', zu einem Gasbeladungsblock 6 abzweigbar, über den, gesteuert von einem Gas-Regelventil 7, Gas G in das flüssige Polyol eintragbar ist. Das derart mit Gas angereicherte Polyol wird in den Polyoltank 2 zurückgeleitet.

Von diesem Polyoltank 2 zweigt eine mit einem steuerbaren Einlassventil 8 versehene Zuführleitung 9 ab, die in ein Messgefäss 10 mündet. Das Messgefäss ist über einen Überlauf 11 mit einem Überlaufgefäss 12 verbunden, dessen Rauminhalt mittels eines Kolbens 13 veränderbar ist. Vom

2

Überlaufgefäss 12 führt eine mit einem steuerbaren Auslassventil 14 versehene Auslassleitung 15 in den Polyoltank 2 zurück. Vom Überlaufgefäss 12 führt ferner eine mit einem steuerbaren Entlüftungsventil 16 versehene Druckluftleitung 17 zu einem 4/3-Wegeventil 18, dessen drei Schaltstellungen mit a, b und c gekennzeichnet sind. Eine weitere Druckleitung 19 führt vom 4/3-Wegeventil 18 zur Unterseite des Kolbens 13. Von den vier gesteuerten Anschlüssen des 4/3-Wegeventils 18 zweigen zwei Anschlüsse zu den Druckleitungen 17 und 19 ab. Die beiden übrigen Anschlüsse führen zu einer Druckluft-Speiseleitung 20 und einer mit einem Dämpfer versehenen Auslassöffnung 21.

Der Kolben 13 ist über eine Kolbenstange 33 mit einem Stellkolben 22 verbunden, dessen Stellung mittels zweier Messfühler 23 und 24 ermittelbar ist. Der Stellkolben 22 ist über ein 4/2-Wegeventil 25 mit Druckluft steuerbar.

Am Boden des Messgefässes 10 ist ein Druck-Messfühler 26, beispielsweise eine Bourdon Dose, ein piezoelektrisches Element oder ein Dehnmessstreifen angeordnet. Dem Druckmessfühler 26 sind ein Verstärker 27, ein Rechner 28 mit einer Speichereinheit und eine Anzeigeneinheit 29 nachgeschaltet. Von der Anzeigeneinheit 29 sind Ausgangssignale einer Vergleicherschaltung 30 zuführbar in der ein gewünschter Sollwert S mit Grenzwerten einstellbar ist, so dass nach Vergleich mit dem von der Anzeigeeinheit 29 ausgegebenen, den Istwert darstellenden Ausgangssignal über eine Steuerleitung 31 ein entsprechender Steuerimpuls zu dem Gasregelventil 7 ergibt, wodurch der Gasbeladungsblock 6 mit Druckgas G beaufschlagbar ist.

Der Kolben 13 weist in der dargestellten Ausführungsform zwei Einrichtungen zum Verstellen auf, von denen die erste von dem 4/3-Wegeventil 18 und den beiden Druckluftleitungen 17 und 19 gebildet wird, und von denen die zweite von dem 4/2-Wegeventil 25 gebildet wird, über das der Stellkolben 22 wechselweise mit Druckluft beaufschlagbar ist.

Das Einlassventil 8, die Ventile 3'' und 3''', das Auslassventil 14, das Entlüftungsventil 16, die Einrichtungen zum Verstellen des Kolbens 13, die von dem 4/3-Wegeventil 18 oder von dem 4/2-Wegeventil 25 steuerbar sind, und die Messleitungen der Messfühler 23 und 24 sowie der Druckmessfühler 26 sind an die Steuereinheit 32 angeschlossen.

Mit der Steuereinheit 32 können die nachfolgend geschilderten Verfahrensschritte durchgeführt werden:

A. Füllen und Spülen des Messzylinders 10.

In der oberen Totpunktlage des Kolbens 13, die über den Messfühler 23 signalisiert wird, ist das Einlassventil 8 geöffnet und das Auslassventil 14 sowie das Entlüftungsventil 16 sind geschlossen. Über die Schaltung des 4/3-Wegeventils 18 in die Schaltstellung b kann die unterhalb des Kolbens 13 befindliche Luft nach aussen abweichen, worauf der Kolben 13 durch den vom Tankvordruck des Polyoltanks 2 vorhandenen Druck zum unteren Totpunkt bewegt wird. Dabei strömt das mit Gas beladene Polyol vom Messgefäss 10 über den Überlauf 11 in das Überlaufgefäss 12. Beim Erreichen der unteren Totpunktlage des Kolbens 13 wird der untere Messfühler 24 aktiviert, worauf über die Steuereinheit 32 das Einlassventil 8 geschlossen und das Auslassventil 14 geöffnet wird. Über die Schaltung des 4/3-Wegeventils 18 in die Schaltstellung G wird der Kolben 13 auf der Unterseite mit Druckluft beaufschlagt, worauf der Kolben 13 nach oben bewegt wird und die im Überlaufgefäss befindliche Menge an Polyol über die Auslassleitung 15 in den Polyoltank 2 zurückfördert. Dieser Vorgang wird solange wiederholt, bis das mit Gas beladene Polyol im Messgefäss 10 vollständig ausgetauscht ist.

Der Vorgang des Füllens und Entleerens der Überlaufgefässes mit damit bewirktem Spülen des Messgefässes kann auch über den Stellkolben 22 erfolgen, der über eine vom 4/2-Wegeventil 25 gesteuerte Druckluft D beaufschlagt werden kann und den Kolben 13 über eine Kolbenstange 33 in der vorbeschriebenen Weise bewegt. Dieser Aufbau erlaubt ein autarkes Arbeiten des Messgerätes.

B. Messung der Gasbeladung der flüssigen Kunststoffkomponente Polyol.

Nach einer von der Steuereinheit 32 gesteuerten Folge von Spülvorgängen wird der Messvorgang eingeleitet, der in der oberen Totpunktstellung des Kolbens 13 beginnt, wobei das Einlassventil 8 und das Auslassventil 14 geschlossen sind. Das 4/3-Wegeventil 18 wird in die Schaltstellung c geschaltet, wodurch der Kolben 13 mittels Druckluft in die untere Totpunktstellung gedrückt wird. Hierauf erfolgt eine Öffnung des Entlüftungsventils 16, so dass die im Messgefäss 10 befindliche Flüssigkeitsmenge Atmosphärendruck ausgesetzt wird. Die mit Gas beladene Kunststoffkomponente dehnt sich aufgrund des Druckabfalls vom Systemdruck auf Atmosphärendruck aus und quillt dabei über den Überlauf 11, der stets für eine konstante Höhe h der im Messgefäss 10 eingeschlossenen Flüssigkeitssäule sorgt.

Nach Abschluss der Entspannungsphase, die in der Regel nach 2 bis 3 Minuten beendet ist, wird, gesteuert von einem Zeitschaltglied, die Dichtemessung mittels des am Boden des Messgefässes 10 angeordneten Druck-Messfühlers 26 vorgenommen. Die Dichte ρ der Flüssigkeitssäule mit der Höhe h bestimmt sich danach nach der Formel

$$\rho = \frac{p}{h \cdot g}$$

wobei p der hydrostatische Druck und g die Erdbeschleunigung darstellen. Da die Werte h und g konstant sind, ist der vom Druck-Messfühler 26 erhaltene Messwert der Dichte der Flüssigkeitssäule direkt proportional.

Der im Druck-Messfühler 26 erhaltene Messwert kann in einem Verstärker 27 noch verstärkt werden und in eine Anzeigeeinheit 29 eingegeben werden, in der die Dichtewerte bzw. Gasbeladungswerte der flüssigen Kunststoffkomponente visuell kontrolliert werden können.

Von der Anzeigeeinheit 29 können die Messwerte zu einer Vergleicherschaltung 30 weitergeleitet werden, in der ein gewünschter Sollwert S mit Grenzwerten einstellbar ist, so dass nach Vergleich mit dem von der Anzeigeeinheit 29 ausgegebenen über eine Steuerleitung 31 ein entsprechender Steuerimpuls zu dem Gasregelventil 7 ergeht, wodurch der Gasbeladungsblock 6 mit dem Gas G zur Gasanreicherung der flüssigen Kunststoffkomponente beaufschlagbar ist.

Der vorbeschriebene Messvorgang kann mit geringem Aufwand noch durch die nachfolgend beschriebene Massnahme beschleunigt werden: Der Druckmessfühler 26 wird bereits zu Beginn der Entspannungsphase geschaltet, worauf in kurzen Zeitabständen Messwerte über den Verstärker 27 einem mit einer Speichereinheit ausgestatteten Rechner 28 zugeführt werden. In diesem Rechner 28 werden die über einen kurzen Zeitraum eingegebenen Werte aufgrund in der Speichereinheit eingespeicherter Bezugswerte, beispielsweise empirisch ermittelter Eichwerte und/oder einer physikalischen Gesetzmässigkeiten entsprechenden mathematischen Funktion, auf einen Endwert hochgerechnet, der sich nach Beendigung der Entspannungsphase einstellt. Dies hat den Vorteil, dass sich das Abwarten auf die Beendigung der Entspannungsphase erübrigt, so dass in kurzer Zeit (ca 15 sec − 30 sec) ein verlässlicher Dichtemesswert zur Verfügung steht.

Eine weitere Massnahme zur Beschleunigung des Messvorgangs kann darin bestehen, dass nach der Einleitung des Messvorgangs bei geschlossenem Einlassventil 8 und Auslassventil 14 der Kolben 13 von der oberen Totpunktstellung nach unten bewegt wird. Die Kolbenbewegung wird dabei über den Stellkolben 22 bewirkt, wobei das 4/2-Wegeventil 25 so geschaltet ist, dass der Stellkolben 22 mit dem Kolben 13 zur unteren Totpunktstellung bewegt wird. Das 4/3-Wegeventil 18 wird in die Schaltstellung b gebracht, so dass sich bei geschlossenem Entlüftungsventil 16 ein Unterdruck im Überlaufgefäss 12 und im Messgefäss 10 einstellt. Durch diesen Unterdruck wird der Entspannungsprozess der mit Gas beladenen Kunststoffkomponente, der bei Atmosphärendruck in der Regel Minuten erfordert, auf ca. 0,5 Minuten verkürzt. Die Dauer der Unterdruckbeaufschlagung wird von einem mit dem Entlüftungsventil 16 gekoppelten Zeitschaltglied bestimmt.

Die vorbeschriebenen Massnahmen und Einrichtungen zur Messung der Gasbeladung einer flüssigen Kunststoffkomponente, wie z.B. Polyol, ermöglichen somit die exakte Bestimmung des Gasanteils in kurzer Aufeinanderfolge, so dass der Gasbeladungsgrad stets in engen Grenzen konstant gehalten werden kann. Damit lassen sich bei der Herstellung von Schaumkunststoffen bestimmte Qualitätseigenschaften des Endprodukts zuverlässig einhalten.

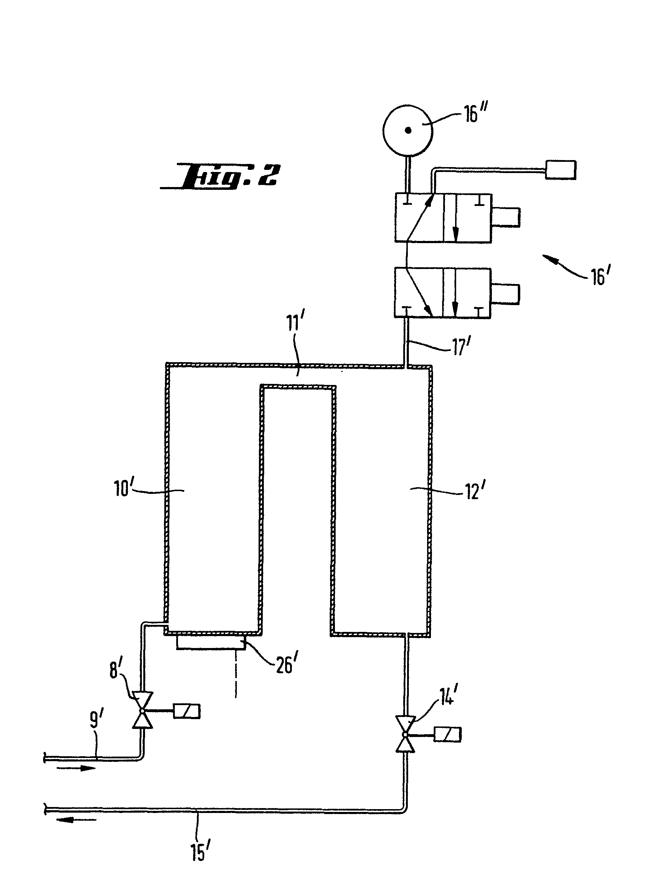

In der Ausführungsform nach Fig. 2 ist ein Messgefäss 10' über eine Zuführleitung 9' an einen Polyoltank (nicht dargestellt) angeschlossen. Die Komponentenzufuhr zum Messgefäss 10' ist über ein Einlassventil 8' steuerbar. Das Messgefäss hat am Boden einen Druckmessfühler 26' angeordnet und ist im oberen Bereich über einen Überlauf 11' mit einem Überlaufgefäss 12' verbunden. Das Überlaufgefäss 12' ist geschlossen und ist an eine Auslassleitung 15' angeschlossen, in der sich ein steuerbares Auslassventil 14' befindet. Im oberen Bereich des Überlaufgefässes 12' ist eine mit einem Schaltventil 16' versehene Druckgasleitung 17' angeschlossen. Das Schaltventil 16' besteht aus einem 2/2 Wegeventil und einem 3/2 Wegeventil über welche der Innenraum des Überlaufgefässes wahlweise absperrbar, mit einer Druckgasquelle 16'' oder mit der Atmosphäre verbindbar ist.

Im Betrieb wird das Messgefäss 10' zunächst gespült, wobei das Auslassventil 14' und das Einlassventil 8' geöffnet und das Schaltventil 16' geschlossen sind. Dabei strömt die flüssige Kunststoffkomponente aus einem Tank (nicht dargestellt) über die Zuführleitung 9' durch das Messgefäss 10' über den Überlauf 11' in das Überlaufgefäss 12' und von dort über die Rückführleitung 15' in den Tank zurück.

Nach dem Spülen des Messgefässes 10' wird die flüssige Kunststoffkomponente aus dem Überlaufgefäss 12' ausgetrieben, wozu das Einlassventil 8' geschlossen und das Auslassventil 14' geöffnet werden und der Innenraum des Überlaufgefässes 12' über das Schaltventil 16' mit der Druckgasquelle 16'' verbunden wird. Die im Überlaufgefäss 12' enthaltene Komponente wird in den Tank zurückgeführt.

Für den anschliessenden Messvorgang werden das Einlassventil 8' und das Auslassventil 14' geschlossen und der Innenraum des Überlaufgefässes über das Schaltventil 16' entlüftet. Nach einer Entspannungsphase wird mittels des Druckmessfühlers 26' der hydrostatische Druck der im Messgefäss 10' enthaltenen Flüssigkeitssäule gemessen und als Mass für die Dichte bzw. den Gasbeladungsgrad der gasbeladenen Kunststoffkomponente ausgewertet. Die Messung ergibt den Gasbeladungsgrad der Komponente bei Atmosphärendruck.

**Patentansprüche**

1. Vorrichtung zur Messung der Gasbeladung einer unter dem Systemdruck einer Anlage zur Herstellung von Schaumkunststoff, insbesondere einer Polyurethan-Schaumanlage, stehenden, flüssigen Kunststoffkomponente, bei der aus einem Tank (2) in periodischen Zeitabständen eine Probemenge in ein Messgefäss (10) abzweigbar ist, in dem mittels eines Überlaufs (11) eine bestimmte Höhe h einer aus flüssiger und gasbeladener Kunststoffkomponente bestehenden Flüssigkeitssäule einstellbar ist, und in dem eine den hydrostatischen Druck p der Flüssigkeitssäule als Mass für die Dichte p der gasbeladenen Kunststoffkomponente wertende Dichtemesseeinrichtung in Form eines Druckmessfühlers angeordnet ist mit

— einem dem Überlauf (11) nachgeordneten Überlaufgefäss (12), dessen Rauminhalt mittels eines Kolbens (13) veränderbar ist,

— Einrichtungen zum Verstellen des Kolbens (13),

— einer an das Messgefäss (10) angeschlossenen, mit einem Einlassventil (8) versehenen Zuführleitung (9),

— einer an das Überlaufgefäss (12) angeschlossenen mit einem Auslassventil (14) versehenen Auslassleitung (15),

— einem mit dem Messgefäss (10) in Verbindung stehenden Entlüftungsventil (16) und

— einer Steuereinheit (32), mit der das Einlassventil (8), das Auslassventil (14), das Entlüftungsventil (16), der Druck-Messfühler (26) und die Einrichtungen zum Verstellen des Kolbens steuerbar sind, wobei die Steuereinheit (32) so programmiert ist, dass zum Zweck der Dichtemessung bei abgesperrten Ein- und Auslassventilen (8, 14) der Rauminhalt des Überlaufgefässes durch Verstellen des Kolbens (13) soweit vergrössert wird, dass sich im Messgefäss (10) durch den Überlauf die bestimmte Höhe h der Flüssigkeitssäule einstellt.

2. Vorrichtung nach Anspruch 1, wobei die Dichtemesseinrichtung aus einem am Boden des Messgefässes (10) angeordneten Druckmessfühler (26) besteht.

3. Vorrichtung nach Anspruch 2, wobei die Dichtemesseinrichtung Einrichtungen zur Kompensation des über der Flüssigkeitssäule herrschenden, vom atmosphärischen Druck abweichenden Druckniveaus aufweist.

4. Vorrichtung nach Anspruch 3, wobei der Dichtemesseinrichtung ein Rechner (28) nachgeschaltet ist, in dem mit Hilfe einiger Anfangs-Messwerte und im Rechner eingespeicherter Bezugswerte, beispielsweise empirisch ermittelter Eichwerte und/oder einer physikalischen Gesetzmässigkeiten entsprechenden mathematischen Funktion, der Dichtewert der gasbeladenen Kunststoffkomponente vor dem Abschluss der Entspannungsphase vorbestimmbar ist.

5. Vorrichtung nach Anspruch 4, wobei die Steuereinheit (32) ein den Öffnungsvorgang des Entlüftungsventils (16) verzögerndes Zeitschaltglied aufweist, wodurch bei abgesperrten Ein- und Auslassventilen (8, 14) und abgesenktem Kolben (13) die Flüssigkeitssäule zwecks Beschleunigung der Entspannungsphase einem Unterdruck aussetzbar ist.

6. Vorrichtung zur Messung der Gasbeladung einer unter dem Systemdruck einer Anlage zur Herstellung von Schaumkunststoff, insbesondere einer Polyurethan-Schäumanlage stehenden, flüssigen Kunststoffkomponente, bei der aus einem Tank (2) in periodischen Zeitabständen eine Probemenge in ein Messgefäss (10′) abzweigbar ist, in dem mittels eines Überlaufs (11′) eine bestimmte Höhe h einer aus flüssiger und gasbeladener Kunststoffkomponente bestehenden Flüssigkeitssäule einstellbar ist, und in dem eine den hydrostatischen Druck p der Flüssigkeitssäule als Mass für die Dichte ρ der gasbeladenen Kunststoffkomponente wertende Dichtemesseinrichtung in Form eines Druckmessfühlers angeordnet ist, und bei der dem Überlauf (11′) ein geschlossenes Überlaufgefäss (12′) nachgeordnet ist, das im unteren Bereich ein steuerbares Auslassventil (14′) und im oberen Bereich ein steuerbares Schaltventil (16′) aufweist, in dessen erster Schaltstellung der Innenraum des Überlaufgefässes (12′) mit einer Druckgasquelle (16″) verbindbar ist und in dessen dritter Schaltstellung der Innenraum des Überlaufgefässes (12′) entlüftbar ist.

7. Vorrichtung nach Anspruch 6, wobei die Dichtemesseinrichtung aus einem am Boden des Messgefässes (10′) angeordneten Druckmessfühler (26′) besteht.

8. Vorrichtung nach Anspruch 7, wobei die Dichtemesseinrichtung Einrichtungen zur Kompensation des über der Flüssigkeitssäule herrschenden, vom atmosphärischen Druck abweichenden Druckniveaus aufweist.

9. Vorrichtung nach Anspruch 8, wobei der Dichtemesseinrichtung ein Rechner (28) nachgeschaltet ist, in dem mit Hilfe einiger Anfangs-Messwerte und im Rechner eingespeicherter Bezugswerte, beispielsweise empirisch ermittelter Eichwerte und/oder einer physikalischen Gesetzmässigkeiten entsprechenden mathematischen Funktion, der Dichtewerte der gasbeladenen Kunststoffkomponente vor dem Abschluss der Entspannungsphase vorbestimmbar ist.

10. Verfahren zum Betrieb der Vorrichtung nach Anspruch 6, bei dem zum Spülen des Messgefässes (10′) das Auslassventil (14′) und das Einlassventil (8′) geöffnet sind und das Schaltventil (16′) geschlossen ist, zum Austreiben der Komponente aus dem Überlaufgefäss (12′) das Einlassventil (8′) geschlossen, das Auslassventil (14′) geöffnet ist und das Schaltventil (16′) den Innenraum des Überlaufgefässes (12′) mit der Druckgasquelle (16″) verbindet und zum Messen der Gasbeladung der im Messgefäss (10′) enthaltenen Flüssigkeitssäule aus gasbeladener Komponente das Einlassventil (14′) geschlossen ist und das Schaltventil (16′) den Innenraum des Überlaufgefässes (12′) mit der Atmosphäre verbindet.

**Claims**

1. Device for measuring the gas charging of a liquid synthetic plastics material component subject to the system pressure of an installation for the production of foam synthetic plastics material, especially a polyurethane foaming installation, in which a sample quantity can be branched off out of a tank (2) at periodical time intervals into a measuring vessel (10) in which a specific height h of a liquid column consisting of liquid and gas-charged synthetic plastics material component is settable by means of an overflow (11), and in which a density-measuring device in the form of a pressure-measuring sensor is arranged which evaluates the hydrostatic pressure p of the liquid column as measure for the density ρ of the gas-charged synthetic plastics material component, having

an overflow vessel (12) arranged after the overflow (11), the volume of which vessel is variable by means of a piston (13), devices for displacing the piston (13),

a supply conduit (9) connected to the measuring vessel (10) and provided with an inlet valve (8),

an outlet conduit (15) connected to the overflow vessel (12) and provided with an outlet valve (14),

an air-discharge valve (16) in communication with the measuring vessel (10) and

a control unit (32) with which the inlet valve (8), the outlet valve (14), the air-discharge valve (16), the pressure-measuring sensor (26) and the devices for displacing the piston are controllable, wherein the control unit (32) is so programmed that for the purpose of measurement of density with inlet and outlet valves shut off the volume of the overflow vessel is enlarged by displacement of the piston (13) to such extent that the specific height h of the liquid column is established by the overflow in the measuring vessel (10).

2. Device according to Claim 1, wherein the density-measuring device consists of a pressure-measuring sensor (26) arranged on the bottom of the measuring vessel (10).

3. Device according to Claim 2, wherein the density-measuring device comprises devices for the compensation of the pressure level, differing from the atmospheric pressure, prevailing over the liquid column.

4. Device according to Claim 3, wherein the density-measuring device is followed by a computer (28) in which, with the aid of several initial measured values and reference values stored in the computer, for example empirically ascertained calibration values and/or a mathematical function corresponding to physical interrelationships, the density value of the gas-charged synthetic plastics material component can be predetermined before the conclusion of the expansion phase.

5. Device according to Claim 4, wherein the control unit (32) comprises a time-switch member delaying the opening operation of the air-discharge valve (16), whereby when the inlet and outlet valves (8, 14) are shut off and the piston (13) is lowered, the liquid column is subjectable to a negative pressure with the purpose of acceleration of the expansion phase.

6. Device for measuring the gas charging of a liquid synthetic plastics material component subject to the system pressure of an installation for the production of foam synthetic plastics material, especially a polyurethane foaming installation, in which a sample quantity can be branched off out of a tank (2) at periodical time intervals into a measuring vessel (10') in which a specific height h of a liquid column consisting of liquid and gas-charged synthetic plastics material component is settable by means of an overflow (11') and in which a density-measuring device in the form of a pressure-measuring sensor is arranged which evaluates the hydrostatic pressure p of the liquid column, as measure for the density ρ of the gas-charged synthetic plastics material component, and in which the overflow (11') is followed by a closed overflow vessel (12') which comprises a controllable outlet valve (12') in the lower region and a controllable change-over valve (16') in the upper region, in the first operational position of which change-over valve the interior of the overflow vessel (12') is connectable with a compressed gas source (16'') and in the third operational position of which change-over valve the interior of the overflow vessel (12') can be discharged of air.

7. Device according to Claim 6, wherein the density-measuring device consists of a pressure-measuring sensor (26') arranged on the bottom of the measuring vessel (10').

8. Device according to Claim 7, wherein the density-measuring device comprises devices for the compensation of the pressure level, differing from atmospheric pressure, which prevails over the liquid column.

9. Device according to Claim 8, wherein the density-measuring device is followed by a computer (28) in which, with the aid of some initial measured values and reference values stored in the computer, for example empirically ascertained calibration values and/or a mathematical function corresponding to physical interrelationships, the density value of the gas-charged synthetic plastics material component is predeterminable before the conclusion of the expansion phase.

10. Process for the operation of the device according to Claim 6, in which for the rinsing of the measuring vessel (10') the outlet valve (14') and the inlet valve (8') are opened and the change-over valve (16') is closed, for the expulsion of the component out of the overflow vessel (12') the inlet valve (8') is closed, the outlet valve (14') is opened and the change-over valve (16') connects the interior of the overflow vessel (12') with the compressed-gas source (16''), and for the measurement of the gas charge of the liquid column, contained in the measuring vessel (10'), of gas-charged component the inlet valve (14') is closed and the change-over valve (16') connects the interior of the overflow vessel (12') with the atmosphere.

**Revendications**

1. Dispositif pour mesurer la teneur en gaz d'un composant liquide générateur de matière plastique, se trouvant sous la pression du système d'une installation de production de mousses de matières plastiques, en particulier une installation de production de mousse de polyuréthanne, dispositif dans lequel, à des intervalles périodiques de temps, une quantité d'échantillon peut être enlevée d'un réservoir (2) pour être dirigée dans un récipient (10) de mesure dans lequel, à l'aide d'un débordement (11), on peut établir une hauteur h déterminée d'une colonne de liquide consistant en du composant, liquide et chargé de gaz, générateur de matière plastique, et dans lequel est disposé, sous forme d'un détecteur de pression, un dispositif de mesure de densité, mesurant la pression hydrostatique p de la colonne de liquide comme mesure de la densité ou masse volumique ρ du composant, générateur de matière plastique et chargé de gaz, ce dispositif comportant

– un récipient (12) de réception du trop-plein, disposé en aval du débordement (11) et dont l'espace intérieur peut être modifié à l'aide d'un piston (13),

– des organes pour déplacer le piston (13),

– un conduit (9) d'acheminement, relié au récipient (10) de mesure et comportant une soupape (8) d'admission,

– un conduit (15) de sortie, relié au récipient (12) et comportant une soupape (14) de sortie,

– une soupape (16) de purge et de dégagement d'air, communiquant avec le récipient (10) de mesure, et

– une unité (32) de commande, pouvant commander la soupape (8) d'admission, la soupape (14) de sortie, la soupape (16) de purge, le détecteur (26) mesureur de pression et les organes destinés à déplacer le piston, l'unité (32) de commande étant programmée de manière que, pour mesurer la masse volumique alors que les soupapes d'admission (8) et de sortie (14) sont fermées, l'espace intérieur du récipient de réception du trop-plein soit agrandi, par déplacement du piston (13), de manière à obtenir dans le récipient (10) de mesure, grâce au débordement, la hauteur h déterminée de colonne de liquide.

2. Dispositif selon la revendication 1, dans lequel l'installation de mesure de densité consiste en un détecteur (26) mesureur de pression, disposé au fond du récipient (10) de mesure.

3. Dispositif selon la revendication 2, dans lequel le dispositif de mesure de la densité présente des organes pour compenser le niveau de pression régnant au-dessus de la colonne de liquide et qui est différente de la pression atmosphérique.

4. Dispositif selon la revendication 3, dans lequel est monté, en aval du dispositif de mesure de densité, un ordinateur (28) dans lequel, à l'aide de certaines valeurs initiales de mesure et de certaines valeurs de référence mises en mémoire dans l'ordinateur, par exemple des valeurs étalonnées déterminées empiriquement et/ou une fonction mathématique correspondant à des lois physiques, on peut déterminer à l'avance, avant la fin de la phase de détente, la valeur de la densité du composant, générateur de matière plastique et chargé de gaz.

5. Dispositif selon la revendication 4, dans lequel l'unité (32) de commande présente un organe temporisateur retardant le processus d'ouverture de la soupape (16) de purge, de sorte que, avec la femeture des soupapes d'admission (8) et de sortie (14) et avec l'abaissement du piston (13), la colonne de liquide peut être soumise à une dépression pour accélérer la phase de détente.

6. Dispositif pour mesurer la teneur en gaz d'un composant liquide générateur de matière plastique se trouvant sous la pression du système d'une installation de production de mousses de matières plastiques, en particulier une installation de production de mousse de polyuréthanne, dispositif dans lequel une quantité d'échantillon peut être prélevée à des intervalles périodiques de temps d'un réservoir (2) et être dirigée dans un récipient (10) de mesure dans lequel, à l'aide d'un débordement (11'), on peut obtenir une hauteur h déterminée d'une colonne de liquide consistant en le composant liquide, générateur de matière plastique et chargé de gaz, et dans lequel est disposé, sous forme d'un détecteur de pression, un dispositif de mesure de densité cotant la pression hydrostatique p de la colonne de liquide comme constituant une mesure de la densité ρ du composant chargé de gaz et générateur de matière plastique, et dans lequel un récipient (12') fermé de réception de trop-plein est placé en aval du débordement (11'), ce récipient présentant, à sa partie inférieure, une soupape (14') commandable de sortie et, dans sa partie supèrieure, un distributeur (16') commandable qui, dans sa première position de commutation peut relier l'espace intérieur du récipient (12) à une source (16'') de gaz comprimé et, dans sa troisième position de commutation, permet de purger et de débarrasser de son air l'espace intérieur du récipient (12') de réception de trop-plein.

7. Dispositif selon la revendication 6, dans lequel le dispositif de mesure de densité consiste en un détecteur (26') mesureur de pression, disposé sur le fond du récipient (10') de mesure.

8. Dispositif selon la revendication 7, dans lequel le dispositif de mesure de densité présente des organes pour compenser le niveau de pression s'écartant de la pression atmosphérique et régnant au-dessus de la colonne de liquide.

9. Dispositif selon la revendication 8, dans lequel, en aval du dispositif de mesure de densité est monté un ordinateur (28) dans lequel, à l'aide de certaines valeurs initiales de mesure et de valeurs de référence mises en mémoire dans l'ordinateur, par exemple des valeurs étalonnées déterminées expérimentalement et/ou une fonction mathématique correspondant à des lois physiques, on peut déterminer à l'avance, avant la fin de la phase de détente, la valeur de la densité du composant chargé de gaz et générateur de matière plastique.

10. Procédé pour la mise en œuvre du dispositif selon la revendication 6, dans lequel, pour balayer et rincer le récipient (10') de mesure, la soupape (14') de sortie et la soupape (8') d'admission sont ouvertes et le distributeur (16') est fermé; pour chasser le composant du récipient (12') de réception du débordement, la soupape (8') d'admission est fermée, la soupape (14') de sortie est ouverte et le distributeur (16') relie l'espace intérieur du récipient (12') avec la source (16'') de gaz comprimé et, pour mesurer la teneur en gaz de la colonne de. liquide contenue dans le récipient (10') de mesure et constituée par le composant chargé de gaz, la soupape (14') d'admission est fermée et le distributeur (16') relie à l'atmosphère l'espace intérieur du récipient (12') de réception du débordement.

Fig. 1

EP 0 125 541 B1

Fig. 2